# EUROPEAN PATENT APPLICATION

(11) **EP 4 495 943 A1**
(43) Date of publication of application: **22.01.2025**
(21) Application number: 24189447.6
(22) Date of filing: 18.07.2024
(51) Int. Cl.: G16H 40/20, G16H 30/20, G16H 20/40, G16H 10/60, G16H 40/63, A61B 5/00, A61F 9/007, G16H 15/00

(54) **COMPUTER IMPLEMENTED METHOD OF GENERATING A PATIENT SPECIFIC DIGITAL WORFKLOW AND AN OPHTHALMIC WORKFLOW GENERATOR FOR GENERATING A PATIENT SPECIFIC DIGITAL WORKFLOW**

(30) Priority: 21.07.2023 CH 7922023
(71) Applicant: Ziemer Ophthalmic Systems AG, 2562 Port (CH)
(72) Inventor: Hutter, Matthias, 3250 Lyss (CH); Keller, Daniel, 3264 Diessbach bei Büren (CH); Marushchenko, Sergii, 2560 Nidau (CH); Rentsch, Sandro, 3008 Bern (CH); Steinlechner, Michael, 8006 Zürich (CH)
(74) Representative: Rentsch Partner AG

(57) **Abstract**

The present disclosure relates to a computer implemented method and to a workflow generation device (110) of generating a patient specific digital workflow of a physical ophthalmic treatment procedure of at least one eye (210) of a patient (200) to be treated. In a processor (112) of a computer system (111), selection data (160) is received (S1) selecting the physical ophthalmic treatment procedure (170). The processor (112) of the computer system (111), determines (S2) resource data of required resources for performing at least one ophthalmic procedural step (171, 172). The processor (112) of the computer system (111) further receives (S3) patient specific ophthalmic data (156) of the eye (210); and generates (S4), the patient specific digital workflow (150) comprising a plurality of interrelating digital workflow items (152) using the determined resource data (154) and the received patient specific ophthalmic data (156).

## Description

### FIELD OF THE DISCLOSURE

The present disclosure relates to a computer implemented method of generating a patient specific digital workflow of a physical ophthalmic treatment procedure of an eye of a patient to be treated, to a computer program product of generating the patient specific digital workflow and to an ophthalmic workflow generator for generating a patient specific digital workflow.

### BACKGROUND OF THE DISCLOSURE

Planning of an eye treatment of a patient, e.g. an eye surgery is a challenging non-linear, multi-step procedure with possible reiterations. In a very basic form, it consists in a first step of entering patient data in a database, in a second step of manually planning one or more diagnostic (pre-operative) measurement sessions involving one or more diagnostic measurement devices. In a further step involving one or more ophthalmic treatment devices and using the entire or only part of the measured diagnostic data, the eye treatment surgery can then be planned manually.

Planning of the individual eye treatment is conventionally performed by a doctor, medical staff or medical personnel. The medical personnel further conventionally maintains and updates the planned individual eye treatment by adding manually additional data of the patient, e.g. by adding measured data of the eye to be treated. The medical staff and the required devices for performing the respective treatment are conventionally manually assigned, such that the eye treatment is feasible at the desired time with the required medical staff and the required devices. In case anything unusual happens, the planned eye treatment may be postponed for several minutes up to several days. The patient, which may be already waiting in the respective room, probably needs to return at another day. Further, maintenance requirements or downtimes of the devices necessary for performing the respective treatment procedure or the availability of medical personnel are conventionally not automatically considered during the planning procedure of the eye treatment. Such planning requires therefore conventionally a lot of effort and individual knowledge of the medical staff performing the planning. Further, parameters / data of the patient, in particular of the eye of the patient, may change, which could require to adapt the planned eye treatment procedure. This further requires a lot of time and energy, in particular because different resource data may have to be collected and updated manually again. The conventional planning process of ophthalmic procedures is therefore in particular time consuming and costly.

### SUMMARY OF THE DISCLOSURE

It is an object of the present disclosure to provide a computer implemented method of generating a patient specific digital workflow of a physical ophthalmic treatment procedure of an eye of the person and an ophthalmic workflow generator for generating the patient specific digital workflow. In particular, it is an object of the present disclosure to provide the computer implemented method of generating the patient specific digital workflow and the ophthalmic workflow generator for generating the patient specific digital workflow, which do not have at least some of the disadvantages of the prior art.

According to the present disclosure, these objects are addressed by the features of the independent claims. In addition, further advantageous embodiments follow from the dependent claims and the description.

According to the present disclosure, a computer implemented method of generating a patient specific digital workflow of a physical ophthalmic treatment procedure of at least one eye of a patient to be treated is specified. The physical ophthalmic treatment procedure is for example a medical procedure for treating the at least one eye of the patient.

The physical ophthalmic treatment procedure may also include and / or may be a diagnostic procedure. For example, the patient specific digital workflow of the physical ophthalmic treatment procedure of one eye of the patient or of both eyes of the patient is generated.

The computer implemented method comprises the step of receiving, in a processor of a computer system, selection data selecting the physical ophthalmic treatment procedure to be performed on the at least one eye of the patient. The selection data comprises information on the specific physical ophthalmic treatment procedure, which is planned to be performed on the at least one specific eye of the patient. A physical ophthalmic treatment procedure is for example a cataract treatment procedure, an eye laser treatment procedure, a subjective refraction measurement procedure, a topography measurement procedure, a wave-front measurement procedure, a combination thereof or any other possible ophthalmic procedure. The selection data is for example received in the processor via a data input interface, transmitted from a user interface, connected to the computer system.

The computer implemented method further comprises the step of determining, by the processor of the computer system, resource data of required resources for performing at least one ophthalmic procedural step of the selected physical ophthalmic treatment procedure. The selected physical ophthalmic treatment procedure may comprise one or several ophthalmic procedural steps, which define or determine the entire physical ophthalmic treatment procedure. The different ophthalmic procedural steps may require different resources necessary for performing the respective steps. The determined resource data may define the required resources, may determine parameter of the required resources or may comprise information of the available resources for performing the at least one ophthalmic procedural step.

The computer implemented method further comprises the step of receiving, in the processor of the computer system, patient specific ophthalmic data of the eye to be treated.

The patient specific ophthalmic data is data of the at least one eye on which the planned physical ophthalmic procedure is to be performed. The ophthalmic data may therefore be uniquely for the respective eye to be treated. The patient specific ophthalmic data may influence the requirements of the selected physical ophthalmic treatment procedure, for example by determining that at least one of the respective steps needs more or less time than average.

The computer implemented method further comprises the step of generating, by the processor of the computer system, the patient specific digital workflow comprising a plurality of interrelating digital workflow items using the determined resource data and the received patient specific ophthalmic data, wherein each of the interrelating digital workflow items represents and determines at least one of the ophthalmic procedural steps of the selected physical ophthalmic treatment procedure of the eye to be treated.

The generated patient specific digital workflow is a digital reference of the selected physical ophthalmic treatment procedure. In other words, the generated patient specific digital workflow is a patient specific digital twin of the selected physical ophthalmic treatment procedure, wherein the digital twin is generated using the determined resource data and the received patient specific ophthalmic data. The digital workflow is at least partially determined by the plurality of interrelating digital workflow items. The digital workflow item are digital references of the different ophthalmic procedural steps forming the selected physical ophthalmic treatment procedure. The interrelating workflow items may influence each other in one or both directions. The method according to the present disclosure enables that a patient specific digital workflow is at least partially automatically created / generated using patient individual data, which reduces time and costs compared to conventional planning. In particular, it is possible generate a digital adaptable patient individual workflow, which is accessible and adaptable from anywhere. The digital workflow is generated using the determined resource data, which are determined/defined in dependence of the selected physical ophthalmic treatment procedure and the digital workflow is further generated using the received patient specific ophthalmic data, data of the eye of the person, which enables that the digital workflow is not only created in dependence of the planned physical ophthalmic treatment but also in dependence of data of the eye, such that a truly patient individual digital workflow is realizable.

In an embodiment, the resource data is determined, by the processor, using received availability data determining the availability of the required resources for performing at least one ophthalmic procedural step of the selected physical ophthalmic treatment procedure. The availability data may comprise occupancy plans of at least one ophthalmic device, which is required for performing the ophthalmic treatment procedure. Further, the availability data may comprise occupancy plans of medical personnel, which is or which are required for performing the ophthalmic treatment procedure. Further, the availability data may comprise data of the patient, determining the timeslots at which the patient may be available for performing at least one of the procedural steps of the selected physical ophthalmic treatment procedure. The availability data is for example stored and updated in a memory, which is accessible by the processor of the computer system.

In an embodiment, the determined resource data comprises device specific data of required ophthalmic diagnostic devices for diagnostic measurement of the eye of the patient, device specific data of required ophthalmic treatment devices for ophthalmic treatment of the eye of the patient, and / or further required resource data required for performing at least one ophthalmic procedural step of the selected physical ophthalmic treatment procedure. The determined resource data comprise for example information on the required ophthalmic diagnostic device type for performing the diagnostic measurement as the at least one ophthalmic procedural step (e.g. a Scheimpflug tomography device) and information on the availability of the specific device type. The device specific data of required ophthalmic treatment device for ophthalmic treatment of the eye of the patient may comprise device type information (e.g. a laser device) and information on the availability of the specific device etc. Further, required resource data may comprise information on the necessary medical personnel for performing the at least one of the procedural steps and information on the availability of the medical personnel or additional information, which has not yet been provided via the availability data.

In a further embodiment, the resource data of required resources is determined, by the processor of the computer system, for each of the ophthalmic procedural steps of the selected physical ophthalmic treatment procedure. Each of the ophthalmic procedural steps of the selected physical ophthalmic treatment procedure may be assigned with the required resource data for performing the respective step.

In an embodiment, the received patient specific ophthalmic data comprises measured data of the eye of the patient measured by an ophthalmic diagnostic device. The patient specific ophthalmic data, e.g. uniquely for each patient, may comprise measured data by an ophthalmic diagnostic device, for example a Scheimpflug tomography device, a Placido topography device and/or other devices, which are configured to measure data of the eye to be treated.

In a further embodiment, the received patient specific ophthalmic data comprises non-measured patient data. The non-measured patient data is preferably received via a human machine interface, which enables that this data is transferred to the processor or made available to the processor. Non-measured patient data is for example data like properties of glasses etc. entered by medical personnel in the human machine interface, which is then made available to the processor.

The patient specific ophthalmic data is in an embodiment received automatically in the processor of the computer system. The processor of the computer system may have access to a memory, which stores the patient specific ophthalmic data, in particular the measured and non-measured data. The memory further may enable that the stored data is updated if necessary, for example in case new measured data is received. The processor may automatically access the memory (database) if triggered. The measured data is for example automatically transmitted from the measurement device to the central memory, which may further synchronize the received new measured data with former stored data.

In an embodiment, the measured data of the eye of the patient is automatically assigned to the respective patient or patient database by the content of the data itself. For example, the measured data comprises a unique pattern of the eye to be treated, which is already stored in the patient database. This unique pattern may trigger e.g. via a positive matching that the newly measured data is automatically assigned to the respective patient or patient database.

In an embodiment, the step of generating the patient specific digital workflow comprises to generate for each ophthalmic procedural step of the selected physical ophthalmic treatment procedure at least one of the interrelating digital workflow item. All of the ophthalmic procedural steps of the selected physical ophthalmic treatment procedure are, according to this embodiment, digitally replicated by one or a plurality of digital workflow items. In another embodiment, only a portion of the ophthalmic procedural steps of the of the selected physical ophthalmic treatment procedure are digitally replicated by one or a plurality of digital workflow items.

In an embodiment, the step of generating of the plurality of interrelating digital workflow items comprises to generate a diagnostic digital workflow item determining a patient specific diagnostic procedural step of the physical ophthalmic treatment procedure for measuring ophthalmic data of the at least one eye of the patient to be treated.

In an embodiment, the step of generating of the plurality of interrelating digital workflow items comprises to generate a treatment digital workflow item determining a patient specific treatment procedural step of the physical ophthalmic treatment procedure for treating the at least one eye of the patient.

In a further embodiment, the step of generating of the plurality of interrelating digital workflow items comprises to generate a post treatment digital workflow item or any other necessary specific digital workflow item for digitally replicating the entire selected physical ophthalmic treatment procedure.

In an embodiment, the diagnostic digital workflow item and / or the treatment digital workflow item or any other digital workflow item comprises action type data, patient parameter data, diagnostic or treatment device specific data, personnel specific data, and / or time specific data. The digital workflow items, which digitally represent at least one ophthalmic procedural step, comprise preferably patient specific data, such that each generated workflow item individually represents the respective patient specific ophthalmic procedural steps. The digital workflow item is for example a data package with connections to other digital workflow items and comprises information on what kind of procedural step it represents. Further it may comprise information on the specific patient, when the physical procedural step is to be performed (timing information) and / or information determining by whom (medical personnel information) and by what specific available device the physical procedural step is to be performed out of the set of potentially available devices.

In an embodiment, the method further comprising the step of transmitting, by the processor, at least one of the generated digital workflow items to at least one of: a diagnostic device, a treatment device or a human machine interface, or any other ophthalmic device connected to the computer system. In another embodiment, the at least one generated digital workflow items are not transmitted, but the processor enables that the ophthalmic devices or the other devices, can access the generated digital workflow items, for example stored in the memory. Both embodiments enable that the generated digital workflow is distributed, in particular its workflow items with the generated data, and made available to the respective diagnostic devices.

In an embodiment, the generated digital workflow items comprise device instructions, which are used by the respective devices for performing the patient specific diagnostic procedural step or the patient specific treatment procedural step or any other patient specific procedural step of the ophthalmic treatment procedure.

In an embodiment, at least one of the interrelating digital workflow items is updated, by the processor of the computer system, using newly received patient specific ophthalmic data, which is captured during the implementation of a temporarily previously arranged ophthalmic treatment procedural step associated with a temporarily previously assigned digital workflow items, and / or wherein at least one of the plurality of the digital workflow items is updated, by the processor, using newly determined resource data and / or newly received availability data. In other words, the generated patient specific digital workflow is preferably updated constantly using newly measured or gathered data / information of the patient, the resources or the physical ophthalmic treatment procedure itself. Other information may further be used to update the digital workflow. The patient specific digital workflow is for example constantly centrally managed, updated and synchronized by the processor of the computer system.

In an embodiment, at least one of the plurality of the interrelating digital workflow items comprises patient individual verification data, uniquely identifying the patient, and wherein the patient individual verification data is configured to be compared with respective data for enabling the execution of the associated ophthalmic treatment procedural step at the respective devices. The execution of the respective ophthalmic treatment procedural step at the respective ophthalmic devices is for example only enabled in case the patient individual verification data is positively matched with the respective data. The patient individual verification data is for example a code stored in the memory and the respective data inserted in the device, or received in the processor, needs to match to the code for enabling the execution of the respective ophthalmic treatment procedural step at the respective device.

In an embodiment, the patient individual verification data comprises at least partially measured diagnostic data of the eye of the patient to be treated, and wherein the patient individual verification data is configured to be compared with measured respective data, in particular measured with the treatment device, for enabling the execution of the associated ophthalmic treatment procedural step. The measured diagnostic data of the eye may uniquely identify the patient, which makes an advantageous verification data. The measured diagnostic data is for example a surface topology of the eye to be treated or an iris color distribution or iris structure of the eye to be treated or any other measured data of the eye to be treated, which is configured to uniquely identify the patient, in particular to uniquely identify the eye to be treated.

In a further embodiment, the method further comprises the step of displaying the generated patient specific digital workflow via a human machine interface device forming part of the computer system or being (at least electronically) connected to the computer system to a user.

In an embodiment, the method further comprises the step of receiving instructions, in the processor of the computer system, from the human machine interface device, which update at least one of the plurality of interrelating digital workflow items of the patient specific digital workflow. The received instructions may comprise information, on which the digital workflow, in particular the interrelating digital workflow items, have to be updated. Such received instructions comprise for example, further information only available to the user of the human machine interface, for example personal preferences of medical personnel or personal preferences of the patient not automatically available to the processor. This can, for example, include information on the patient needing special assistance or personalized notes to medical personnel.

In an embodiment, the computer system is configured to use a digital imaging and communications standard for data transfer between the different devices and as data storage standard, for example in the memory. The standard is for example the Digital Imaging and Communications in Medicine (DICOM) standard. This enables that further medical data management systems may access the computer system, such that the patient specific digital workflow and / or gathered data can be advantageously shared.

In an embodiment, the computer system is configured to use the DICOM standard for data transfer between the different devices and as data storage standard, for example in the memory, by mapping work items to Unified Procedure Steps as defined by the DICOM Standards Committee.

In a further embodiment, the method being further configured to generate a plurality of patient specific digital workflows, the method comprises:
- receiving, in the processor of the computer system, a plurality of selection data selecting the physical ophthalmic treatment procedure to be performed on eyes for a plurality of patients;
- determining, by the processor of the computer system, resource data of required resources for performing at least one ophthalmic procedural step of each of the selected physical ophthalmic treatment procedures;
- receiving, in the processor of the computer system, patient specific ophthalmic data of the eyes to be treated;
- generating, by the processor of the computer system, the plurality of patient specific digital workflow each comprising a plurality of interrelating digital workflow items using the determined resource data and the received patient specific ophthalmic data, wherein each of the interrelating digital workflow items represents and determines at least one of the ophthalmic procedural steps of the plurality of selected physical ophthalmic treatment procedure of the eyes to be treated.

According to a further aspect, a computer program product of generating a patient specific digital workflow of a physical ophthalmic treatment procedure of at least one eye of a patient to be treated is specified. The computer program product comprising computer program code configured to direct a processor of a computer system such that the processor performs the following steps:
- receive selection data selecting the physical ophthalmic treatment procedure to be performed on the at least one eye of the patient;
- determine, resource data of required resources for performing at least one ophthalmic procedural step of the selected physical ophthalmic treatment procedure;
- receive patient specific ophthalmic data of the at least one eye to be treated;
- generate the patient specific digital workflow comprising a plurality of interrelating digital workflow items using the determined resource data and the received patient specific ophthalmic data, wherein each of the interrelating digital workflow items represents and determines at least one of the ophthalmic procedural steps of the physical ophthalmic treatment procedure of the eye to be treated.

In a further embodiment, the computer program code is configured to control the processor to perform a method as described above and hereinafter.

According to a further aspect of the present disclosure, an ophthalmic workflow generator for generating a patient specific digital workflow of a physical ophthalmic treatment procedure of at least one eye of a patient to be treated is specified. The ophthalmic workflow generator comprising a computer system comprising at least one processor, the at least one processor being configured to perform the following steps:
- receive selection data selecting the physical ophthalmic treatment procedure to be performed on the at least one eye of the patient;
- determine, resource data of required resources for performing at least one ophthalmic procedural step of the selected physical ophthalmic treatment procedure;
- receive patient specific ophthalmic data of the at least one eye to be treated;
- generate the patient specific digital workflow comprising a plurality of interrelating digital workflow items using the determined resource data and the received patient specific ophthalmic data, wherein each of the interrelating digital workflow items represents and determines at least one of the ophthalmic procedural steps of the physical ophthalmic treatment procedure of the at least one eye to be treated

In a further embodiment, the ophthalmic workflow generator is further configured to perform a method as described above and hereinafter.

### BRIEF DESCRIPTION OF THE DRAWINGS

The herein described disclosure will be more fully understood from the detailed description given herein below and the accompanying drawings, which should not be considered limiting to the invention described in the appended claims. The drawings in which:
- **Figure 1**: shows a schematic view of an ophthalmic workflow system according to a first exemplary embodiment;
- **Figure 2**: shows a schematic view of a physical ophthalmic treatment procedure and a respective patient specific digital workflow according to a first embodiment;
- **Figure 3**: shows a schematic view of different method steps of a method of generating the patient specific digital workflow;
- **Figure 4**: shows a first detailed schematic view of a portion of the method steps of the method of Figure 3;
- **Figure 5**: shows a second detailed schematic view of the method steps of the method of Figure 3;
- **Figure 6**: shows a third detailed schematic view of the method steps of the method of Figure 3;
- **Figure 7**: shows a generated patient specific digital workflow displayed to a user on a display of the human machine interface in a matrix according to a first exemplary embodiment;
- **Figure 8**: shows a generated patient specific digital workflow comprising diagnostic digital workflow items displayed in a matrix;
- **Figure 9**: shows a generated patient specific digital workflow comprising treatment digital workflow items displayed in a matrix;
- **Figure 10**: shows the matrix of Figure 9, in particular showing the interrelation of the different workflow items;
- **Figure 11**: shows a plurality of generated patient specific digital workflows for a plurality of patients displayed to a user on a display of the human machine interface according to an exemplary embodiment.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

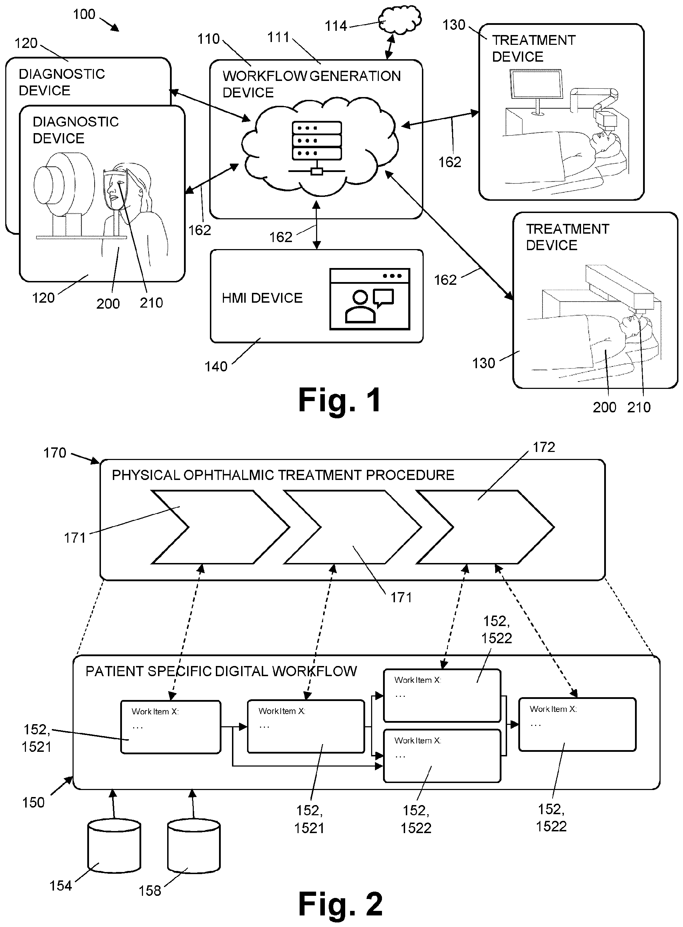

**Figure 1** schematically illustrate a schematic view of an ophthalmic workflow system 100 according to a first exemplary embodiment. The ophthalmic workflow system 100 comprises a workflow generation device 110, a plurality of diagnostic devices 120, a plurality of treatment devices 130 and a human machine interface device 140. The ophthalmic workflow system 100 is according to this embodiment further connected to a local or remote server system 114 via a specific connection, for example using a DICOM-standard network connection. This connection may enable the ophthalmic workflow system 100 to be connected to the internet, an intranet or other servers or devices. The workflow generation device 110 is for example a central server, a cloud server a local workstation, a mobile device, any other kind of electronic device, or a combination of the aforementioned. The human machine interface 140 may form part of the workflow generation device 110 or is a separate device, for example, a workstation, a notebook a mobile device, a handheld device, the diagnostic device 120 itself, the treatment device 130 itself or any other device connected or being capable to interact with the workflow generation device 110. The different devices are according to this embodiment connected with each other and in particular with the workflow generation device 110 via a data transfer connection 162. The data transfer connection 162 is for example a wired and / or a wireless connection, using any kind of telecommunication standard.

The diagnostic devices 120 are for example devices configured to perform diagnostic measurements. Such diagnostic devices 120 are, for example, Scheimpflug tomography devices, Placido topography devices, optical biometry devices, wavefront analyzers, autorefractometers, OCT imaging devices, tonometers or retinal imaging devices.

The treatment devices 130 are for example devices configured to perform an ophthalmic treatment step. Such treatment devices 120 are, for example, laser systems, in particular laser systems for refractive surgery, for cataract surgery and more general for therapeutic applications on corneal, lens or retinal diseases or eye conditions.

The ophthalmic workflow system 100, is for example installed in an ophthalmic clinic, in an ophthalmic department of a hospital, in a medical practice or in any other medical ophthalmic facility. The connection of the different devices 120, 130, 140 with the workflow generation device 110 enables to control and to determine the different possible ophthalmic treatment procedures performed on one or both eyes 210 of a patient 200.

The workflow generation device 110 comprises a processor 111 or a plurality of processors 111, which are configured to perform calculations, based on received or determined data, the processor may be an electronic circuit, an application specific integrated circuit (ASIC), a field programmable gate array (FPGA), an integrated circuit or a computer chip or a combination of the aforementioned.

**Figure 2** shows a schematic view of a physical ophthalmic treatment procedure 170 and a respective patient specific digital workflow 150 according to a first embodiment. The physical ophthalmic treatment procedure 170 is the medical procedure, which the patient 200 goes through during his respective individual treatment. The physical ophthalmic treatment procedure 170 is for example a laser-assisted in situ keratomileusis (LASIK) treatment, a cataract surgery treatment or any other treatment procedure and / or diagnostic procedure involving ophthalmology. The physical ophthalmic treatment procedure 170 may involve one or a plurality of patient specific diagnostic procedural steps, 171, 172, for example at least one diagnostic procedural step 171 and / or at least one treatment procedural step 172. The diagnostic procedural step 171 comprises for example the step of measuring the eye 210 of the patient 200 using one of the diagnostic devices 120. The treatment procedural step 172 comprises for example the step of treating the eye 210 of the patient 200 using one of the treatment devices 130. The physical ophthalmic treatment procedure 170, may comprise additional procedural steps, for example, post treatment steps or prior diagnostic steps, or additional steps in between. The entirety of the patient specific procedural steps 171, 172 determine / define the physical ophthalmic treatment procedure 170 of the eye 210 to be treated, for example from entering the ophthalmic facility till a final post-surgical check.

**Figure 2** further shows a patient specific digital workflow 150 comprising a plurality of interrelating digital workflow items 152. The patient specific digital workflow 150 is a digital copy, a digital twin of the physical ophthalmic treatment procedure 170. In other words, the patient specific digital workflow 150 represents the individual physical ophthalmic treatment procedure 170 in a digitalized manner. The patient specific digital workflow 150 comprises therefore the interrelating digital workflow items 152, which represent at least partially one of the patient specific diagnostic procedural steps 171. Figure 2 shows the interrelationship between the different digital workflow items 152 schematically via arrows. Some of the workflow items 1521 represent a diagnostic procedural step 171 and some of the workflow items 1522 represent a treatment procedural step 172, a combination is also conceivable. Figure 2 further shows that the patient specific digital workflow 150 uses resource data 154 and availability data 158 for determining the specific digital workflow items 152. The patient specific digital workflow 150 is generated, maintained and updated by the processor 112 of the computer system 111 of the ophthalmic workflow generator 110. The patient specific digital workflow 150 is further displayed to a user of the human machine interface 140 and the patient specific digital workflow 150 may be updated due to user input received via the human machine interface 140. Generating, maintaining and updating of the patient specific digital workflow 150 for one or a plurality of patient will be explained in more detail below.

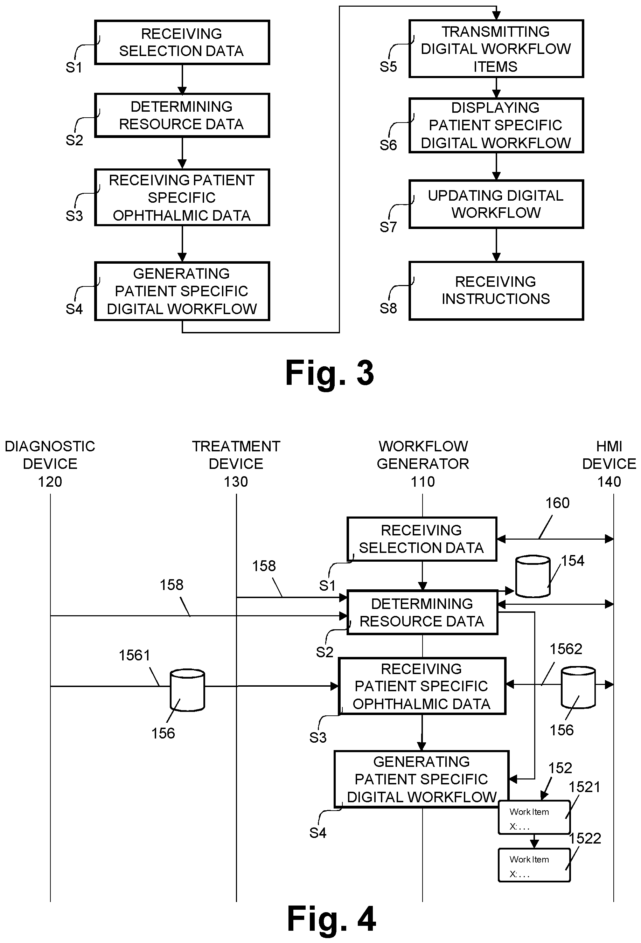

**Figure 3** shows a schematic view of different method steps of a method of generating the patient specific digital workflow 150. The computer implemented method shown in Figure 3 is for example at least partially executed in one or the plurality of the processors 112 of the computer system 111 of the ophthalmic workflow generator 110 of the ophthalmic workflow system 100. In the following paragraphs, described with reference to the Figures 3 to 6 are possible sequences of steps performed by the computer system 111 and / or its one or more processors 1112 for generating the patient specific digital workflow 150.

As illustrated in the **Figures 3** to **6****,** in step S1, the processor 112 of the computer system 111 receives S1 selection data 160 selecting the physical ophthalmic treatment procedure 170 to be performed on the eye 210 of the patient 200. The selection data 160 is for example the selected physical ophthalmic treatment procedure 170 out of a plurality of available physical ophthalmic treatment procedures 170. The selection data 160 is for example received from the human machine interface 140 used by medical personnel or the patient 200 itself. The available physical ophthalmic treatment procedures 170 are for example stored in the processor 112 or a memory and presented to the medical personnel or the user, preferably via the human machine interface 140, for an advantageous simple selection procedure.

As illustrated in the **Figures 3** to **6****,** in step S2, the processor 112 of the computer system 111 determines S2 resource data 154 or required resources for performing at least one ophthalmic procedural step 171, 172 of the selected physical ophthalmic treatment procedure 170. It is preferred that the determined resource data 154 is displayed via the human machine interface device 140 to the user, as indicated in Figure 4 via the respective arrow. The resource data 154 may define the required resources, may determine parameter of the required resources or may comprise information of the available resources for performing the at least one ophthalmic procedural step 171, 172.

As illustrated in the **Figures 3** to **6****,** in step S3, the processor 112 of the computer system 111 receives S3 patient specific ophthalmic data 156 of the eye 210 to be treated. The patient specific ophthalmic data 156 is data of the at least one eye 210 or of both eyes 210 on which the selected physical ophthalmic procedure 170 is to be performed. The ophthalmic data 156 may therefore be uniquely for the respective eye 210 to be treated and / or uniquely for both respective eyes 210 to be treated of the patient 200. The patient specific ophthalmic data 156 may influence the requirements / parameter of the selected physical ophthalmic treatment procedure 170, in particular of the respective ophthalmic procedural steps 171, 172, for example, by determining that at least one of the respective steps 171, 172 probably needs more or less time than average.

As illustrated in the **Figures 3** to **6****,** in step S4, the processor 112 of the computer system 111 generates S4 the patient specific digital workflow 150 comprising a plurality of interrelating digital workflow items 152 using the determined resource data 154 and the received patient specific ophthalmic data 156. Each of the interrelating digital workflow items 152 represents and determines preferably at least one of the ophthalmic procedural steps 171, 172 of the physical ophthalmic treatment procedure 170 of the eye 210 to be treated. The patient specific digital workflow 150 is uniquely generated for the individual patient 200 in dependence of boundary conditions defined by the determined resource data, like treatment devices 130 and diagnostic devices 120 necessary for performing the physical treatment procedure 170, and by the patient specific ophthalmic data 156 of the eye(s) 210 of the patient 200. In other words, the automatically generated patient specific digital workflow 150 takes into account patient data and resource data, which makes the patient specific digital workflow 150 in particular accurate and useful for planning and performing the physical ophthalmic treatment procedure 170.

As illustrated in the **Figures 3** and **4** to **6,** in step S5, the processor 112 of the computer system 111 transmits S5 at least one of the generated digital workflow items 152 to at least one of: a diagnostic device 120, a treatment device 130 or a human machine interface 140, connected to the computer system 111. Additionally or alternatively, the at least one generated digital workflow items 152 are made available to the ophthalmic diagnostic 120 or treatment devices 130 or the other devices 140, such that they can access the generated digital workflow items 152, for example stored in the memory. Both embodiments enable that the generated digital workflow 152 is distributed, in particular its workflow items 152 with the generated data, is made available to the respective devices of the ophthalmic workflow system 100. For example, the ophthalmic workflow system 100 comprises a plurality of diagnostic devices 120 and a plurality of treatment devices 130. The generated digital workflow items 152 are transmitted only to one of the diagnostic devices 120, which has been determined to perform the respective diagnostic procedural step. Alternatively, the digital workflow item 152 is only made available to the one diagnostic device 120, which has been determined to perform the respective diagnostic procedural step. The same applies mutatis mutandis to the treatment devices 130 or to any other of the devices of the ophthalmic workflow system 100.

As illustrated in the **Figures 3** and **4** to **6,** in step S6, the processor 112 of the computer system 111 displays S6 the patient specific digital workflow 150 to a user of the ophthalmic workflow system 100. The user may be the patient 200 and / or medical personnel. It is of course also conceivable that the entire process of generating the patient specific digital workflow 150 is displayed from the beginning till the end to the user. For example, the selection (step S1) of the physical ophthalmic treatment procedure 170 is displayed, the determined (step S2) resource data 154 is displayed, the received (step S3) patient specific ophthalmic data 156 is displayed and the further steps may also be constantly (updated) displayed to the user. Advantageously, the synchronization status between the devices in the workflow system 100 is also displayed to the user, in particular when the diagnostic devices 120, the treatment device 130, and / or the human machine interface 140 of the workflow system 100 are located remotely and accessed via a network connection.

As illustrated in the **Figures 3** and **4** to **6,** in step S7, the processor 112 of the computer system 111, updates S7 at least one of the interrelating digital workflow items 152 and / or the patient specific digital workflow 150 using newly received patient specific ophthalmic data 156, which is captured during the implementation of a temporarily previously arranged ophthalmic treatment procedural step 171, 172 associated with temporarily previously assigned digital workflow items 152, and / or wherein at least one of the plurality of the digital workflow items 152 is updated S7, by the processor 112, using newly determined resource data 154 and / or newly received availability data 158. According to this step, generated patient specific digital workflow 150 is at least once, preferably constantly updated due to new data or new information gathered or measured during the implementation of an ophthalmic treatment procedural step 171, 172 of the ophthalmic treatment procedure 170. For example, the generated patient specific digital workflow 150 is updated due to newly received patient specific data 156 measured during the implementation of one diagnostic ophthalmic procedural step 171, in particular e.g. during the refractive measurement of the eye 210 of the patient 200. The results of the measurements may substantiate, that the previously estimated time for performing the respective treatment procedural step 172 requires more time or may need to be performed using another treatment device 130 and / or by different medical personnel. It is of course also conceivable that input data from the patient 200, for example a change in his personal schedule, may require to update the patient specific digital workflow 150. The patient 200 or medical personnel may have access, e.g. via an application on his smartphone, to the computer system 111 and may send respective input data 190 to the computer system 111, preferably via a wireless network, and may receive a respective notification, that the patient specific digital workflow 150 has been updated due to its input data190. Updating of the patient specific digital workflow 150 may trigger sending of calendar invitations / updating of calendar invitations to the patient 200 or the medical personnel.

As illustrated in the **Figures 3** and **4** to **6,** in step S8, the processor 112 of the computer system 111, receives S8 input data 190, for example via a directly updated receives digital workflow item 152, or via the HMI device 140 or any other device. The input data 190 is received according to this embodiment from the diagnostic device 120 or the treatment device 130. The received new input data 190 may be displayed to the user and may automatically update the generated patient specific digital workflow 150. The Figures show schematically a plurality of steps S7 and S8, thereby indicating that the generated patient specific digital workflow 150 may be constantly updated S7 in dependence of different kind of instructions or data received.

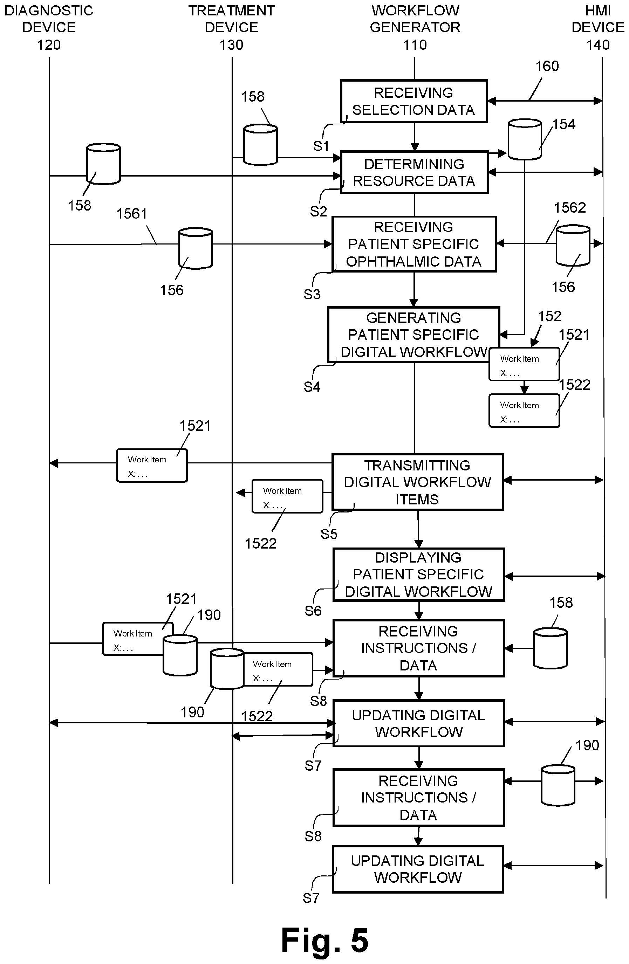

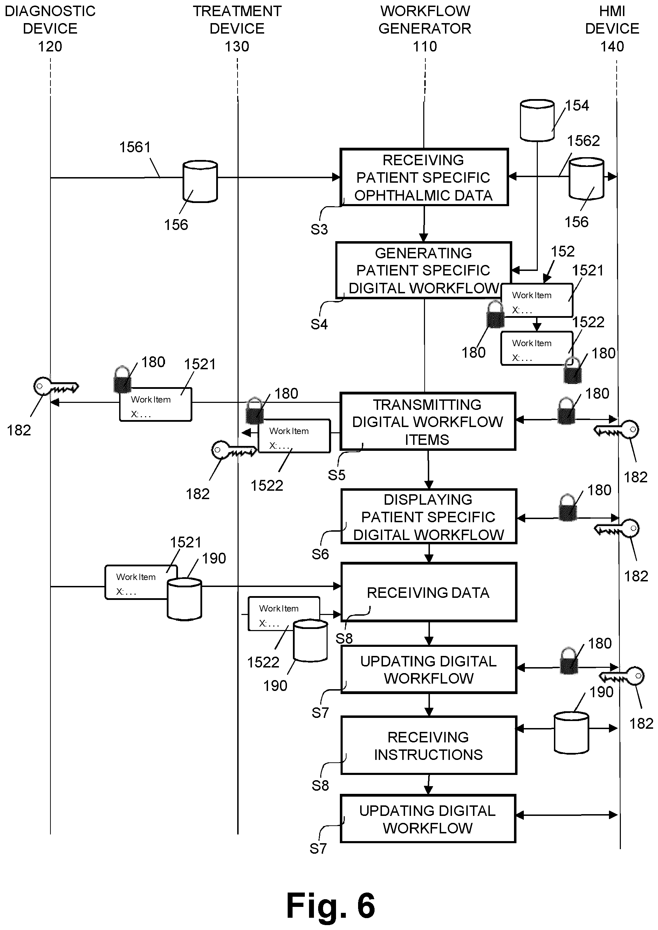

**Figure 6** shows a third detailed schematic view of the method steps of the method of generating the patient specific digital workflow 150. Figure 6 differs from Figure 5 at least that in step S4 patient individual verification data 180 is additionally generated during the process of generating the patient individual workflow 150 (step S4). The patient individual verification data 180 may be generated randomly or using received patient specific data 156. The patient individual verification data 180 is for example measured data, captured by a diagnostic device 120. The patient individual verification data 180 could be topology data of the eye 210 or color distribution data of the eye 210 or any other data, which may uniquely identify the eye 210 of the patient 200. It is of course also conceivable that the patient individual verification data 180 could be a code or any other (randomly) generated data, which enables to uniquely identify the patient 200. The patient individual verification data 180 may be associated with the generated digital workflow 150 and / or with specific generated digital workflow items 152, such that different digital workflow items 152 may have different verification data 180. In a further embodiment, the patient individual verification data 180 may be determined in dependence of the determined resource data 154 (step S2). For example, the verification data 180 is automatically generated in dependence of the specific medical personnel, which has been determined in step S2 to perform the respective patient specific procedural step 171, 172. In this case, only the specific medical personnel may has the corresponding key, positively matching to the verification data 180, unlocking for example respective devices 120, 130, for performing the patient specific procedural step 171, 172. Figure 6 further shows that the respective digital workflow items 152 with the verification data 180 are transmitted to the respective devices 120, 130 140 or are made accessible for the respective devices 120, 130, 140. For example, the patient 200 may only see his generated digital workflow 150 on his mobile device 140 if he enters key data 182, which positively matches with the verification data 180. The same may apply to medical personnel.

The respective diagnostic devices 120 or treatment devices 130 may for example only be enabled to perform the respective patient specific treatment procedural step 171, 172 if the entered key data 182 positively matches with the respective verification data 180. For example, the verification data 180 for performing the patient specific treatment procedural step 172 with the respective treatment device 130 comprises measured data of the eye 210 of the patient 200. The respective treatment device 130 may have the capability to measure the same data of the eye 210 of the patient 200 to be treated. In this case, the measured data of the treatment device 130 is the key data 182, which needs to positively match with the verification data 180, which is the previously measured data of the eye 210.

**Figure 6** further shows that the each interaction with the patient specific digital workflow 150 may only be enabled if the respective key data 182 positively matches with the respective verification data 180.

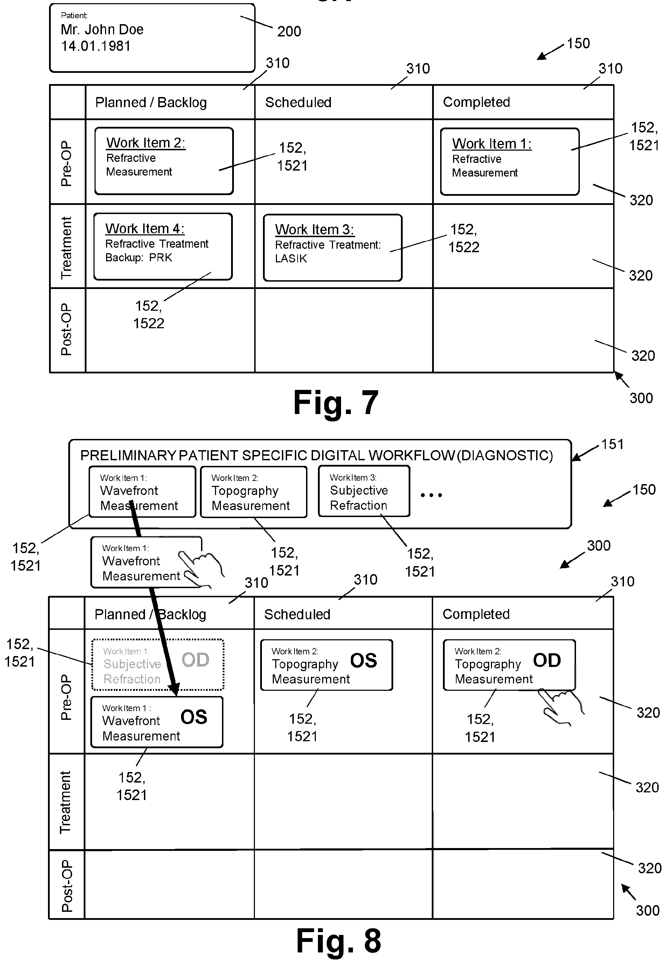

**Figure 7** shows a generated patient specific digital workflow 150 of one patient displayed to a user on a display of the human machine interface 140 according to a first exemplary embodiment. The patient specific digital workflow 150 is presented in a user interface matrix 300. The matrix 300 according to this embodiment comprises three columns 310. A first column 310 for planned / backlog work items 152, a second column 310 for scheduled work items 152 and a third column 310 for completed work items 152. The matrix 300 according to this embodiment further comprises three rows 320. A first row 320 for work items 152 relating to pre operational procedural steps 171, a second row 320 for work items 152 relating to treatment procedural steps 172 and a third row 320 for work items 152 relating to post operational procedural steps of the physical ophthalmic treatment procedure 170. In another embodiment, the matrix 300 may comprise additional and / or different rows 320, and / or additional and / or different columns 310. The matrix 300 advantageously helps to visualize the patient specific digital workflow 150.

According to the embodiment as shown in **Figure 7****,** the patient specific digital workflow 150 for the patient 200, Mr. John Doe, comprises four workflow items 152. The first workflow item 152 relating to a refractive measurement 1521 has already been completed. The second workflow item 152 relating to an additional refractive measurement 1521, for example by a different diagnostic device 120, is planned but not yet scheduled or completed. Figure 7 further shows two treatment digital workflow items 1522 arranged in the respective treatment row 320. The third work item 152 relates and represents a refractive treatment LASIK step 1522 and is scheduled. The fourth work item 152 relates and represents a refractive treatment backup step 1522. The work item 4 is for example a backup work item 152, in case the third work item 152 is not executable as scheduled. According to the embodiment as shown in Figure 7, the classification of the different workflow items 152 in the matrix 300 may be performed automatically and is for example even part of the step of generating S4 the patient specific digital workflow 150. The step of updating S7 of the digital workflow 150 may include to automatically shift one of the workflow items 152 from one column 310 into another column 310. Other visual amendments in dependence of the different steps and their implementation, like changing the color etc. are of course also conceivable. Data received from the respective devices 120, 130 may automatically update the user interface matrix 300, for example in that the respective workflow item 150 is moved from one column 310 to another column 310.

**Figure 8** shows a generated patient specific digital workflow 150 of one patient 200 displayed to a user on a display of the human machine interface 140 according to a second exemplary embodiment. This embodiment differs from the embodiment as shown in Figure 7 in that a preliminary patient specific digital workflow 151 (relating to a diagnostic treatment procedures 170) comprising a preliminary sequence of the digital workflow items 152, is automatically generated. The preliminary patient specific digital workflow 151 is for example generated in dependence of the selection data. 160. The different preliminary generated digital workflow items 152 are not automatically classified in the user interface matrix 300, as previously described with respect to Figure7. Figure 8 indicates that the user of the human machine interface 140, for example, medical personnel may move the generated different workflow items 152 from the preliminary patient specific digital workflow 151 into the respective cell of the matrix 300. Moving one specific workflow item 152 into one specific cell of the matrix 300 may automatically trigger to move the other workflow items 152 into the matrix 300, such an automatically filling may be confirmed by the medical personnel. In another embodiment, the medical personnel needs to move each of the workflow items 152 separately into the matrix. The automatically or manually filling may be assisted automatically in dependence of the determined resource data 154, patient specific ophthalmic data 156, availability data 158 or other input data 190. This partially automated process may be in particular advantage in case aspects of planning of the physical ophthalmic treatment procedure 170 are not entirely available to the processor 112. It is also conceivable that a portion of the generated digital workflow items 152 is automatically filled in the matrix 300. Further, in another embodiment, the different digital workflow items 152 are for example automatically prefilled in the matrix 300 and the user of the human machine interface device 140 may confirm or amend the prefilled position of the different digital workflow items 152.

**Figure 8** and the further Figures comprise the abbreviation OS, which stands for oculus sinister (left eye 210 of the patient 200) and the abbreviation OD, which stands for oculus dexter (right eye 210 of the patient 200). The embodiment as shown in Figure 8 comprises the patient specific digital workflow 150 for both eyes 210 of the patient 200. The patient specific digital workflow 150 as shown in Figure 8 comprises diagnostic digital workflow items 1521, the treatment digital workflow items 1522 (not shown in Figure 8) may be generated later.

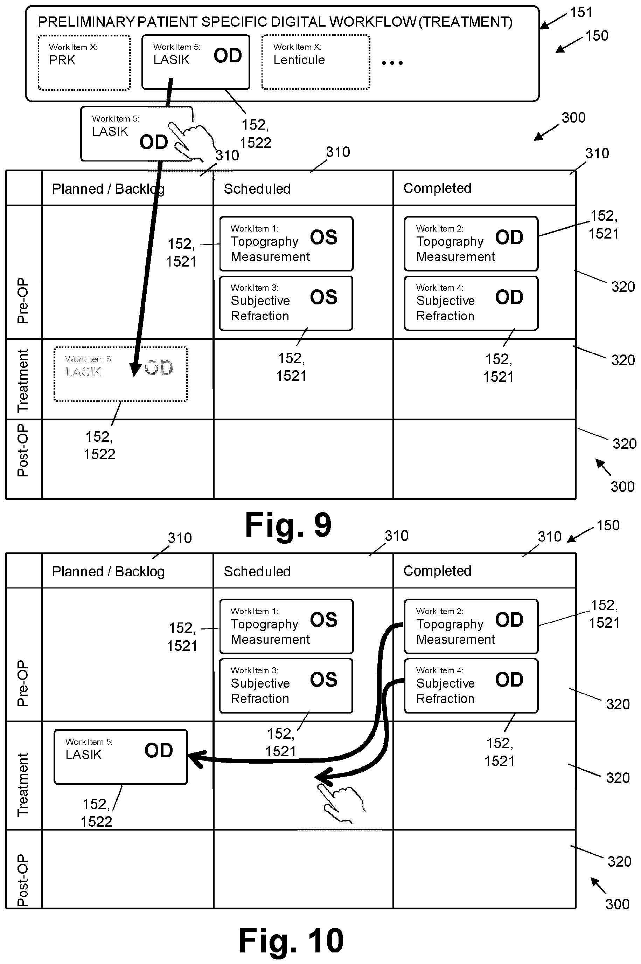

**Figure 9** shows a generated patient specific digital workflow 150 to a treatment procedure 170 of the eyes 210 of the patient 200. Figure 9 shows that the generated patent specific digital workflow 150 comprises a treatment digital workflow item 1522 relating to a LASIK treatment of the right eye 210 of the patient 200. Figure 9 shows that the respective generated workflow item 1522 may be classified automatically or manually in the matrix 300, as explained with respect to Figure 8. Figure 9 further shows that the diagnostic digital workflow items 1521 relating to the right eye 210 are already completed and enable therefore that the respective treatment workflow item 1522 is generated / updated and / or positioned (manually or automatically) in the respective row 320 of the matrix 300. Figure 9 additionally shows that the diagnostic digital workflow items 1521 relating to the left eye 210 of the patient 200 are in the "scheduled" column 310 of the matrix 300, which means that the respective patient specific diagnostic procedural steps 171 are not yet completed, after their completion the respective digital workflow items 1521 relating to the left eye 210 may be automatically or manually shifted into the completed column 310 of the matrix 300, which automatically triggers for example that the further digital treatment workflow items 1522 are generated or enabled for manual placement. In an embodiment, enabling or disabling placing at least one of the digital work flow items 152 is realized by grey out / or allow to place but then marking them with signal color that shows missing data/input.

**Figure 10** further shows the matrix 310 of Figure 9 after successful placing of the fifth treatment digital work flow item 1522. Showing in particular the interrelations of the different workflow items 152. The interrelation between the second workflow item 1521 and the fifth workflow item 1522 is for example automatically generated. The data measured during the topography measurement is for example automatically transmitted and / or used for the determination of the fifth workflow item 1522. Figure 10 further shows that the interrelation between the fourth workflow item 1521 and the fifth workflow item 1522 is for example established manually, for example, by the user of the human machine interface device 140. In this case, the data measured during the implementation of the patient specific diagnostic procedural step 171 (subjective refraction) relating to the fourth digital workflow item 1521 may additionally be used in the determination of the fifth workflow item 1522. In a further embodiment, the interrelations between the work items is visualized with arrows, lines or similar and can be shown on demand.

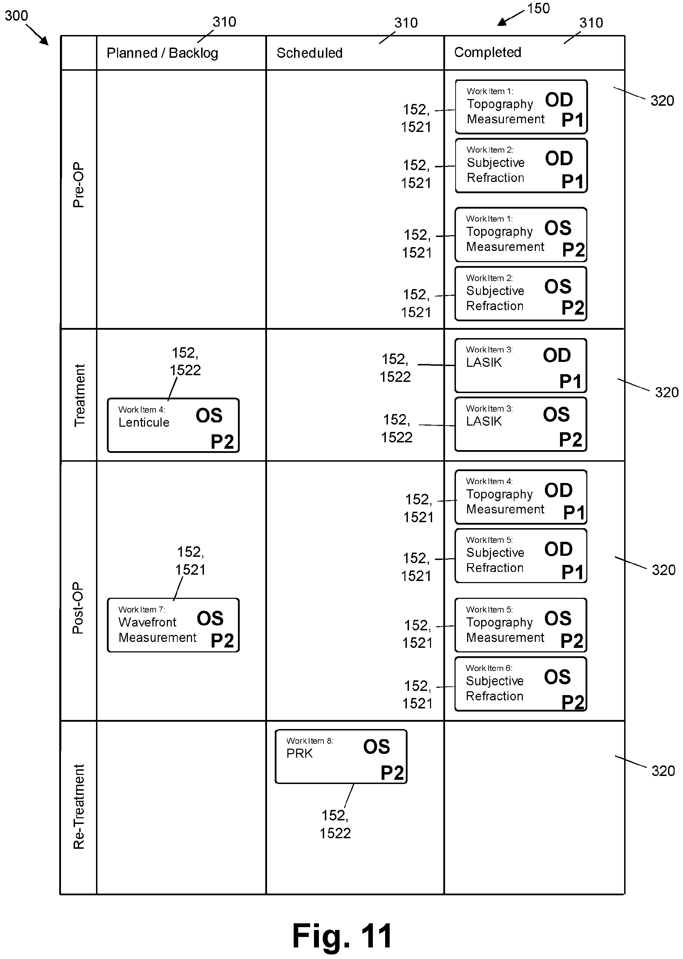

**Figure 11** shows a plurality of generated patient specific digital workflows 150 for a plurality of patients 200 displayed to a user on a display of the human machine interface 140 according to an exemplary embodiment. Figure 11 in particular shows a generated patient specific digital workflow 150 comprising a plurality of digital workflow items 152 for a first patient 200, and a generated patient specific digital workflow 150 comprising a plurality of digital workflow items 152 for a second patient 200. The workflow items 152 relating to the first patient 200 are marked with P1 and the workflow items 152 for the second patient 200 are marked with P2. Figure 11 further shows that the eye 210 to be treated of the first patient P1 is the right eye 210 (OD) and that the eye 210 to be treated of the second patient P2 is the left eye 210 (OS).

The patient specific digital workflow 150 of the first patient 200 (P1) comprises a first diagnostic digital workflow item 1521, relating to the patient specific diagnostic procedural step 171 of topography measurement (Pre-OP), a second diagnostic digital workflow item 1521, relating to the patient specific diagnostic procedural step 171 of subjective refraction (Pre-OP), a third treatment digital workflow item 1522, relating to the patient specific treatment procedural step 172 of LASIK (treatment), a fourth diagnostic digital workflow item 1521, relating to the patient specific diagnostic procedural step 171 of topography measurement (Post-OP) and a fifth diagnostic digital workflow item 1521, relating to the patient specific diagnostic procedural step 171 of subjective refraction (Post-OP).

The patient specific digital workflow 150 of the second patient 200 (P2) comprises a first diagnostic digital workflow item 1521, relating to the patient specific diagnostic procedural step 171 of topography measurement (Pre-OP), a second diagnostic digital workflow item 1521, relating to the patient specific diagnostic procedural step 171 of subjective refraction (Pre-OP), a third treatment digital workflow item 1522, relating to the patient specific treatment procedural step 172 of LASIK (treatment), a fourth treatment digital workflow item 1522, relating to the patient specific treatment procedural step 172 of Lenticule treatment (treatment row), a fifth diagnostic digital workflow item 1521, relating to the patient specific diagnostic procedural step 171 of topography measurement (Post-OP) and a sixth diagnostic digital workflow item 1521, relating to the patient specific diagnostic procedural step 171 of subjective refraction (Post-OP), a seventh diagnostic digital workflow item 1521, relating to the patient specific diagnostic procedural step 171 of wavefront measurement (Post-OP). This workflow item 152 is in the planned column 310, indicating that the respective diagnostic procedural step 171 has not yet been scheduled or performed. The patient specific digital workflow 150 of the second patient further comprises an eight treatment workflow item 1522, relating to the patient specific treatment procedural step 172 of photorefractive keratectomy (PRK) (In the Re-Treatment row 320). The eight digital workflow item 152 is scheduled but not yet completed, which might indicate that this will be the next procedural step 172 of the physical ophthalmic treatment procedure 170 for the second patient.

**Figure 11** advantageously shows that the pre-OP workflow items 152 of both patients 200, namely the topography measurement 1521 and the subjective refraction measurement 1521 of the first patient, and the topography measurement 1521 and the subjective refraction measurement 1521 of the second patient 2000 are already in the completed column 310 of the matrix 300, which means that the respective patient specific diagnostic procedural step 171 has already been completed. The matrix 300 advantageously enables to visualize the progress of the different physical ophthalmic treatment procedures 170 of different patients 200. For example, after the completion of one of the procedural steps 171, 172, the respective workflow items 1521, 1522 are shifted (automatically or manually) into the next column 310, which may trigger generating and / or implementation of the respective next workflow item 152 of the respective digital workflow 150. Figure 11 further advantageously shows that several patient specific digital workflows 150 can be displayed to the user such that he always has the overview of the different stages of the patient specific digital workflows 150.

It should further be noted that, in the description, the sequence of the steps has been presented in a specific order, one skilled in the art will understand, however, that the order of at least some of the steps could be altered, without deviating from the scope of the disclosure.

### LIST OF REFERENCE SYMBOLS

- 100: Ophthalmic workflow system
- 110: Ophthalmic workflow generator
- 111: Com puter system
- 112: Processor
- 114: Remote server system
- 120: Diagnostic device
- 130: Treatment device
- 140: Human machine interface device
- 150: Patient specific digital workflow
- 151: preliminary patient specific digital workflow
- 152: Digital workflow item
- 1521: Diagnostic digital work flow item
- 1522: Treatment digital work flow item
- 154: Resource data
- 1541: Device specific data of required ophthalmic diagnostic devices
- 1542: Device specific data of required ophthalmic treatment devices
- 1543: Further required resource data
- 156: Patient specific ophthalmic data
- 1561: Measured patient data
- 1562: Non-measured patient data
- 158: Availability data
- 160: Selection data
- 162: Data transfer connection
- 170: Physical ophthalmic treatment procedure

- 171: Patient specific diagnostic procedural step
- 172: Patient specific treatment procedural step
- 180: Patient individual verification data
- 182: Key data
- 190: Input data
- 200: Patient
- 210: Eye of the patient
- 300: User interface matrix
- 310: Matrix column
- 320: Matrix row
- S1: Receiving selection data
- S2: Determining resource data
- S3: Receiving patient specific ophthalmic data
- S4: Generating the patient specific digital workflow
- S5: Transmitting digital workflow item
- S6: Displaying patient specific digital workflow
- S7: Updating patient specific digital workflow
- S8: Receiving instructions

## Claims

1. A computer implemented method of generating a patient specific digital workflow (150) of a physical ophthalmic treatment procedure (170) of at least one eye (210) of a patient (200) to be treated, the method comprising:
a. receiving (S1), in a processor (112) of a computer system (111), selection data (160) selecting the physical ophthalmic treatment procedure (170) to be performed on the at least one eye (210) of the patient (200);
b. determining (S2), by the processor (112) of the computer system (111), resource data (154) of required resources for performing at least one ophthalmic procedural step (171, 172) of the selected physical ophthalmic treatment procedure (170);
c. receiving (S3), in the processor (112) of the computer system (111), patient specific ophthalmic data (156) of the at least one eye (210) to be treated;
d. generating (S4), by the processor (112) of the computer system (111), the patient specific digital workflow (150) comprising a plurality of interrelating digital workflow items (152) using the determined resource data (154) and the received patient specific ophthalmic data (156), wherein each of the interrelating digital workflow items (152) represents and determines at least one of the ophthalmic procedural steps (171, 172) of the physical ophthalmic treatment procedure (170) of the at least one eye (210) to be treated.

2. The method according to claim 1, wherein the resource data (154) is determined (S2), by the processor (112), using received availability data (158) determining the availability of the required resources for performing at least one ophthalmic procedural step (171, 172) of the selected physical ophthalmic treatment procedure (170).

3. The method according to one of the preceding claims, wherein the resource data (154) comprises at least one of: device specific data of required ophthalmic diagnostic devices (1541) for diagnostic measurement of the at least on eye (210) of the patient (200), device specific data of required ophthalmic treatment devices (1542) for ophthalmic treatment of the at least one eye (210) of the patient (200), or further required resource data (1543) required for performing at least one ophthalmic procedural step (171, 172) of the selected physical ophthalmic treatment procedure (170).

4. The method according to one of the preceding claims, wherein the received patient specific ophthalmic data (156) comprises measured data (1561) of the at least one eye (210) of the patient (200), which is measured by an ophthalmic diagnostic device (120).

5. The method according to one of the preceding claims, wherein the received patient specific ophthalmic data (156) comprises non-measured patient data (1562) received via a human machine interface (140), which is configured to provide a non-measured patient data input possibility.

6. The method according to one of the preceding claims, wherein generating (S3) of the plurality of interrelating digital workflow items (152) comprises to generate:
a. a diagnostic digital workflow item (1521) determining a patient specific diagnostic procedural step (171) of the physical ophthalmic treatment procedure (170) for measuring ophthalmic data (156) of the at least one eye (210) of the patient (200) to be treated; and / or
b. a treatment digital workflow item (1522) defining a patient specific treatment procedural step (172) of the physical ophthalmic treatment procedure (170) for treating the at least one eye (210) of the patient (200).

7. The method according to claim 6, wherein the diagnostic digital workflow item (1521) and / or the treatment digital workflow item (1552) and / or any other generated digital workflow item comprises at least one of: action type data, patient parameter data, diagnostic or treatment device specific data, personnel specific data, or time specific data.

8. The method according to one of the preceding claims, the method further comprising the step of transmitting (S5) at least one of the generated digital workflow items (152) to at least one of: a diagnostic device (120), a treatment device (130) or a human machine interface (140), connected to the computer system (111).

9. The method according to claim 8, wherein the generated digital workflow items (152) comprise device instructions, which are used by the respective devices (120, 130, 140) for performing the patient specific diagnostic procedural step (171) or the patient specific treatment procedural step (172) of the patient specific ophthalmic treatment procedure (170).

10. The method according to claim 9, wherein at least one of the interrelating digital workflow items (152) is updated (S7), by the processor (112) of the computer system (111), using newly received patient specific ophthalmic data (156), which is captured during the implementation of a temporarily previously arranged ophthalmic treatment procedural step (171, 172) associated with a temporarily previously assigned digital workflow items (152), and / or wherein at least one of the plurality of the digital workflow items (152) is updated (S7), by the processor (112), using newly determined resource data (154) and / or newly received availability data (158).

11. The method according to one of the claims 9 or 10, wherein at least one of the plurality of the interrelating digital workflow items (152) comprises patient individual verification data (180), uniquely identifying the patient (200), and wherein the patient individual verification data (180) is configured to be compared with respective data for enabling the execution of the associated ophthalmic treatment procedural step (172) at the respective devices (120, 130, 140).

12. The method according to claim 11, wherein the patient individual verification data (180) comprises at least partially measured diagnostic data of the at least one eye (210) of the patient (200) to be treated, and wherein the patient individual verification data (180) is configured to be compared with measured respective data, in particular measured with the treatment device (130), for enabling the execution of the associated ophthalmic treatment procedural step (172).

13. The method according to one of the preceding claims, wherein the method further comprises the step of displaying (S6) the generated patient specific digital workflow (150) via a human machine interface device (140) forming part of the computer system (111) or being connected to the computer system (111) to a user.

14. The method according to claim 13, wherein the method further comprises the step of receiving instructions (S8), in the processor (112) of the computer system (111), from the human machine interface device (140), which update at least one of the plurality of interrelating digital workflow items (152) of the patient specific digital workflow (150).

15. A computer program product of generating a patient specific digital workflow (150) of a physical ophthalmic treatment procedure (170) of at least one eye (210) of a patient (200) to be treated, the computer program product comprising computer program code configured to direct a processor (112) of a computer system (110) such that the processor (112) performs the following steps:
a. receive (S1) selection data (160) selecting the physical ophthalmic treatment procedure (170) to be performed on the at least one eye (210) of the patient (200);
b. determine (S2), resource data (154) of required resources for performing at least one ophthalmic procedural step (171, 172) of the selected physical ophthalmic treatment procedure (170);
c. receive (S3) patient specific ophthalmic data (156) of the at least one eye (210) to be treated;
d. generate (S4) the patient specific digital workflow (150) comprising a plurality of interrelating digital workflow items (152) using the determined resource data (154) and the received patient specific ophthalmic data (156), wherein each of the interrelating digital workflow items (152) represents and determines at least one of the ophthalmic procedural steps (171, 172) of the physical ophthalmic treatment procedure (170) of the at least one eye (210) to be treated.

16. The computer program product of claim 15, wherein the computer program code is further configured to control the processor (112) to perform a method according to one of the claims 1 to 14.

17. Ophthalmic workflow generator (110) for generating a patient specific digital workflow (150) of a physical ophthalmic treatment procedure (170) of at least one eye (210) of a patient (200) to be treated, the ophthalmic workflow generator (110) comprising a computer system (111) comprising a processor (112), the processor (112) being configured to perform the following steps:
a. receiving (S1) resource data (154) of required resources for performing at least one ophthalmic procedural step (171, 172) of the physical ophthalmic treatment procedure (170);
b. receiving (S2), patient specific ophthalmic data (156) of the at least one eye (210) to be treated;
c. generating (S3), a plurality of interrelating digital workflow items (152) using the received resource data (154) and the received patient specific ophthalmic data (156), wherein each of the interrelating digital workflow items (152) represents and determines at least one of the ophthalmic procedural steps (171, 172) of the physical ophthalmic treatment procedure (170) of the at least one eye (210) to be treated, thereby generating the patient specific digital workflow (150).

18. The ophthalmic workflow generator (110) of claim 17 being further configured to perform a method according to any one of the claims 1 to 14.
